# EUROPEAN PATENT SPECIFICATION

(11) **EP 3 909 539 B1**
(45) Date of publication and mention of the grant of the patent: **07.06.2023**
(21) Application number: 20787301.9
(22) Date of filing: 21.02.2020
(51) Int. Cl.: A61B 34/30, A61G 13/10, A61B 17/56, B25J 5/02, A61B 90/50, A61B 90/57

(54) **DETACHABLE AND COLLAPSIBLE DOUBLE-GANTRY MECHANICAL ARM SURGICAL ROBOT**
CHIRURGIEROBOTER MIT ABNEHMBAREM UND FALTBAREM MECHANISCHEM DOPPELPORTALARM
ROBOT CHIRURGICAL À BRAS MÉCANIQUE À DOUBLE PORTIQUE AMOVIBLE ET PLIABLE

(30) Priority: 11.04.2019 CN 201910287445; 25.04.2019 CN 201910338027; 10.07.2019 CN 201910619039
(43) Date of publication of application: 17.11.2021
(73) Proprietor: Suzhou Dianhe Medical Technology Co., Ltd., Suzhou, Jiangsu 215000 (CN)
(72) Inventor: ZHANG, Chunlin, Suzhou, Jiangsu 215000 (CN); ZHANG, Xiaokai, Suzhou (CN)
(74) Representative: Petculescu, Ana-Maria
(86) International application number: PCT/CN2020/076149
(87) International publication number: WO 2020/207123

(56) References cited:
- CN-A- 109 259 968
- CN-B- 106 821 666
- CN-B- 107 019 613
- US-A1- 2009 308 400
- US-A1- 2018 028 387

## Description

### TECHNICAL FIELD

The present invention belongs to the technical field of intelligent medical treatments, a surgical robot design, and more particularly, relates to a detachable and collapsible double-gantry mechanical arm surgical robot.

### BACKGROUND

In recent years, some orthopedic surgical robots have been successful in clinic facilities, such as the Mazor Spine Assist in Israel, the SPINEBOT spin robot in South Korea, and the Mazor X robot for spine surgery, and the Think robot for j oint surgery in the United States and so on. Some of these robots are fixed on spinous processes of a human body, but mostly adopt a single-arm structure, which faces the following problems: 1. it has a light load, for example, a spine surgical robot fixed on the spinous process of the human body obviously cannot be overloaded, and a wheeled single-arm joint-type robot installed on a trolley also belongs to a light-load robot; 2. it has poor rigidity, for example, the wheeled single arm joint-type spine surgical robot has a mechanical arm with a very long cantilever structure, which can meet requirements when a distal end is merely designed to bear one guide tube to guide the placement of a pedicle screw, but if the distal end of the mechanical arm needs to bear a relatively heavy instrument, it is easily deformed which affects the accuracy of surgery; 3. it is difficult to further improve the accuracy of the system due to the aforementioned structures, so that improvement of the operation accuracy of the robot encounters a bottleneck problem, and it is difficult to further improve the accuracy; 4. a function is singular, and it is mostly difficult for the single mechanical arm structure to expand and upgrade, which affects the implementation of multi-functional orthopedic surgeries; 5. it is huge in volume, so that patients tend to be intimidated: some orthopedic surgical robots are large in structure, which easily cause patients to fear and generate certain psychological pressure; and 6. it occupies more space in an operating room, which affects the convenience of operation. CN109259968A discloses a medical auxiliary bed for cardiac operation which comprises a base, two knife-edge fixing portions, four hand and foot fixation parts and a cutting part, and wherein the patient is first placed on the bedplate, the positions of the four L-bars are adjusted by means of the four splints, hand-foot fix portions control by four first hydraulic cylinders are positioned above that patient's hands and feet, the patient's limbs are fixed by two arc clamps through the coordinated work of the hand and foot fixing parts, the second sliding frame at the leftmost end of the base slides to drive the cutting part to find a suitable cutting position, and then control the scalpel to operate on the patient, driving the two knife edge fixing portions to move to both sides of the knife cutting position through the second sliding frames on both sides of the base, the skin on both sides of the knife edge is sucked by suction cups on both sides, and the knife edge is extended by two knife edge fixing parts.

The information disclosed in the Background merely aims to increase the understanding of the overall background of the present invention, and should not be regarded as confirmation or imply that the composition of the information has been commonly known prior art in any form for those ordinarily skilled in the art.

### SUMMARY

In view of the above-mentioned shortcomings, the applicant actively researches and innovates to provide a detachable and collapsible double-gantry mechanical arm surgical robot to make it more valuable in industry.

In order to achieve the above purpose, the present invention provides a detachable and collapsible double-gantry mechanical arm surgical robot and includes an operating table having a longitudinal dimension, a transversal dimension and a vertical dimension. X-direction guidance control devices are arranged in pairs on both longitudinal sides of the operating table. Main swing arms are connected to the operating table through a main connecting base installed on the X-direction guidance control devices. A main push rod is installed on the main connecting base. The main push rod is connected to a lower end of the main swing arm, and pushes the main swing arm to rotate. The main connecting base and an upper end of the main swing arm are connected to a main positioning lock. The main swing arm is further connected to a Y-direction guidance device, and the Y-direction guidance device is connected to a Z-direction guidance control device, thereby forming a main gantry mechanical arm. Main positioning lock (5) locks and supports the main gantry mechanical arm.

Secondary swing arms are connected to the operating table through a secondary connecting base installed on the X-direction guidance control devices, and a cross beam is connected between the secondary swing arms through a quick-change mechanism, thereby forming a secondary gantry mechanical arm.

A secondary push rod is connected between the secondary connecting base and the secondary swing arm. The secondary push rod is connected to a lower end of the secondary swing arm and pushes the secondary swing arm to rotate. A secondary positioning lock for increasing positional rigidity of the secondary swing arm is arranged between the secondary swing arm and the secondary connecting base such that the secondary positioning lock locks and supports the secondary gantry mechanical arm; wherein the longitudinal dimension of the operating table is defined as X-direction; the transversal dimension of the operating table is defined as Y-direction and, the vertical dimension of the operating table (1) is defined as Z-direction; X, Y, Z reference frame formed by the X-direction, Y-direction, Z-direction is orthogonal. The main swing arm rotates around a main swing axis parallel to the Y-direction. Secondary swing arm rotates around a secondary swing axis parallel to the Y-direction.

Preferably, in the above technical solutions, the X-direction guidance control device includes an X-direction guide rail. A main X-direction sliding block and a secondary X-direction sliding block are arranged on the X-direction guide rail. The main X-direction sliding block is connected to the main connecting base. The secondary X-direction sliding block is connected to the secondary connecting base. An X-direction motion motor is arranged at one end of the X-direction guide rail.

Preferably, in the above technical solutions, the top end of the main swing arm is connected to the Y-direction guidance device through an opening and closing device. The opening and closing device includes a positioning sleeve. The positioning sleeve is provided with a mounting hole, and a lock nut is arranged in the mounting hole in a penetrating manner. The positioning sleeve is installed at the top of the main swing arm through a lock bolt. A positioning boss is arranged on the positioning sleeve, a groove is arranged at a corresponding position of the top of the main swing arm, and the positioning boss is embedded in the groove.

Preferably, in the above technical solutions, the main swing arm is provided with a main accommodating groove for accommodating the main positioning lock, and the secondary swing arm is provided with a secondary accommodating groove for accommodating the secondary positioning lock.

Preferably, in the above technical solutions, each of the main positioning lock and the secondary positioning lock includes a connecting sleeve. An upper connecting rod is inserted in an upper end of the connecting sleeve, and a lower connecting rod is inserted in a lower end of the connecting sleeve. An upper end of an interior of the connecting sleeve is provided with a main right-hand thread, and a lower end of the interior of the connecting sleeve is provided with a main left-hand thread. The portion of the upper connecting rod inserted in the connecting sleeve is provided with a main right-hand thread, and the portion of the lower connecting rod inserted in the connecting sleeve is provided with a main left-hand thread. A nut with forward and reverse rotations is arranged in the connecting sleeve.

Preferably, in the above technical solutions, a connecting buckle is arranged at the top of the secondary swing arm, and the cross beam performs positioning and connecting through the connecting buckle.

Preferably, in the above technical solutions, the Y-direction guidance device includes a Y-direction drive motor. A rotating shaft is installed on the Y-direction drive motor. The main pushing element is connected between the main connecting base and the main swing arm.

By adopting the above technical solutions, the present invention has at least the following advantages:
1. The main gantry mechanical arm and the secondary gantry mechanical arm are independent from each other, and instruments are quickly switched through the quick-change structures installed on the Z-direction drive motor and the cross beam, which can implement coordination of multiple surgical instruments and different surgical operations. Moreover, the double-arm configuration is adopted, where the main gantry mechanical arm has the optimal bearing capacity and the secondary gantry mechanical arm has the flexible adjustment range. The two arms cooperate with each other to achieve high-precision synchronous operation similar to the operation performed by two hands, which overcomes various shortcomings of the prior single-arm operation, so as to satisfy different operation requirements of a variety of surgeries, have good scalability and adapt to surgical requirements of multiple disciplines.
2. Independent X-direction, Y-direction and Z-direction guidance control devices are arranged, which can cooperate with subsequent control software, so as to control and adjust the multi-axis motion, and improve the accuracy of the surgical operation of the robot.
3. The main gantry mechanical arm and the secondary gantry mechanical arm both can be separately disassembled and assembled, which facilitates the installation of the sterile jacket and ensures the safety and sterility of implementation.
4. The independent push rod structure is adopted, which can provide effective support and height adjustment for each gantry mechanical arm, so as to satisfy the requirements of various kinds of surgeries. After disassembly, the volume is significantly reduced to avoid excessive occupation of the space in the operating room.
5. The overall configuration is simple, which can be matched with various kinds of surgical instruments to easily satisfy requirements for multi-functional expansion of the robot.

Preferably, in the above technical solutions, a laser ranging sensor is installed on the Z-direction guidance control device. A joint fixing device is installed on the cross beam. The joint fixing device includes a fixing plate. A level gauge is installed on the fixing plate. The fixing plate is simultaneously connected to a main coordinate acquisition needle and a secondary coordinate acquisition needle.

Preferably, in the above technical solutions, the fixing plate is in a U shape. The fixing plate is provided with a fixing hole, and a fixing nail is connected in the fixing hole. A main fixing rod and a secondary fixing rod are arranged on the fixing plate, respectively. The main coordinate acquisition needle is installed on the main fixing rod, and the secondary coordinate acquisition needle is connected on the secondary fixing rod.

Preferably, in the above technical solutions, each of the main coordinate acquisition needle and the secondary coordinate acquisition needle includes an accommodating cannula. A plurality of needle bodies are distributed in array in the accommodating cannula. A shaft-through structure is arranged at a side of the accommodating cannula.

Preferably, in the above technical solutions, the Z-direction guidance control device includes a Z-direction drive motor, and a quick-change mechanism is installed on the Z-direction drive motor.

By adopting the above technical solutions, the present invention has at least the following advantages:
1. The main gantry mechanical arm and the secondary gantry mechanical arm are independent from each other. The secondary gantry mechanical arm has the flexible adjustment range and has a good function of fixing bones (lower limbs such as femur and tibia). The main gantry mechanical arm has the optimal bearing capacity, and quickly switches the instruments through the quick-change structures installed on the Z-direction drive motor and the cross beam. Through the mutual cooperation of the configuration of the two arms, where one firmly grasps and the other flexibly cuts, the high-precision synchronous operation similar to the operation performed by two hands can be achieved, which greatly improves the accuracy of j oint surgery.
2. Independent X-direction, Y-direction and Z-direction guidance control devices are arranged, which can cooperate with subsequent control software, so as to control and adjust the multi-axis motion, improve the accuracy of the surgical operation of the joint replacement robot and expand the scope of application.
3. The main gantry mechanical arm and the secondary gantry mechanical arm both can be separately disassembled and assembled, which facilitates the installation of the sterile jacket and ensures the safety and sterility of implementation.
4. The main coordinate acquisition needle, the secondary coordinate acquisition needle and the laser ranging sensor cooperate with each other to map the bone surface coordinates to the outside of the body, which facilitates providing an accurate position reference, so that the influence of periosteum coverage, bleeding, body fluid and others on the acquisition of the bone coordinate points during the joint replacement surgery can be ideally avoided.

Preferably, in the above technical solutions, a rotating drum instrument library and rotary drive mechanism is installed on the secondary gantry mechanical arm, and includes a rotating drum. A rotating table is arranged at the bottom of the rotating drum. An orthopedic power lifting mechanism, a soft tissue cutting tool lifting mechanism and a forceps lifting mechanism are arranged in the rotating drum, respectively. An upper end of the rotating drum forms a motor chamber, and a lower end of the rotating drum forms a transmission chamber.

Preferably, in the above technical solutions, the orthopedic power lifting mechanism includes a first motor located in the motor chamber. A lower end of the first motor is connected to a first guide rail located in the transmission chamber. A first sliding block is installed on the first guide rail, and an orthopedic power connector is installed on the first sliding block.

Preferably, in the above technical solutions, the soft tissue cutting tool lifting mechanism includes a second motor located in the motor chamber. A lower end of the second motor is connected to a second guide rail located in the transmission chamber. A second sliding block is installed on the second guide rail, and a soft tissue cutting tool connector is installed on the second sliding block.

Preferably, in the above technical solutions, the forceps lifting mechanism includes a third motor located in the motor chamber. A lower end of the third motor is connected to a third guide rail located in the transmission chamber. A third sliding block is installed on the third guide rail, and a forceps connector is installed on the third sliding block.

By adopting the above technical solutions, the present invention has at least the following advantages:
1. The main gantry mechanical arm and the secondary gantry mechanical arm are independent from each other, and the instruments are quickly and automatically switched through a quick-change structure of the rotating drum instrument library, so as to implement the high-precision coordinated operation similar to the operation performed by two hands and even multiple hands.
2. The present invention has large bearing capacity and good rigidity. Since the gantry structure has the relatively strong rigidity and a very short cantilever, various shortcomings of the prior long cantilever joint-type robots are overcome to install a variety of surgical instruments while implementing heavy load, so as to satisfy different operation requirements of various kinds of spinal surgeries.
3. Independent X-direction, Y-direction and Z-direction guidance control devices are arranged, which can cooperate with subsequent control software, so as to implement multi-axis motion control and adjustment and implement separately independent control of orthopedic instruments, forceps instruments and soft tissue cutting tools, thereby improving the flexibility and accuracy of the surgical operation of the robot.
4. The main gantry mechanical arm and the secondary gantry mechanical arm both can be separately disassembled and assembled, which facilitates the installation of the sterile jacket and ensures the safety and sterility of implementation.
5. The independent push rod structure is adopted, which can provide effective support and height adjustment for each gantry mechanical arm, so as to satisfy the requirements of various kinds of surgeries. After disassembly, the volume is significantly reduced to avoid excessive occupation of the space in the operating room, improve the operation convenience and reduce the patient's psychological fear.
6. The rotating drum instrument library has good scalability, which can satisfy requirements of various kinds of spinal surgeries and even requirements of surgeries of other disciplines.

### BRIEF DESCRIPTION OF THE DRAWINGS

FIG. 1 is a side view of the structure of a detachable and collapsible double-gantry mechanical arm surgical robot according to Embodiment 1.
FIG. 2 is a side view of the structure of a main gantry mechanical arm according to Embodiment 1.
FIG. 3 is a side view of the structure of a surgical robot in a use state according to Embodiment 2 (taking a knee arthroplasty as an example, including contours of lower limbs).
FIG. 4 is a top view of the structure of the surgical robot in the use state according to Embodiment 2 (taking a knee arthroplasty as an example, including contours of lower limbs).
FIG. 5 is a schematic diagram of the structure of a main coordinate acquisition needle according to Embodiment 2.
FIG. 6 is a schematic diagram of installation of a rotating drum instrument library and rotary drive mechanism with other surgical instruments according to Embodiment 3.
FIG. 7 is a side view of the structure of the rotating drum instrument library and rotary drive mechanism according to Embodiment 3.
FIG. 8 is a front view of the structure of the rotating drum instrument library and rotary drive mechanism according to Embodiment 3.

Meanings of respective reference signs in the figures are as follows:

| | | | |
|---|---|---|---|
| 1 | operating table | 2 | main connecting base |
| 3 | main swing arm | 4 | Y-direction guidance device |
| 5 | main positioning lock | 6 | secondary swing arm |
| 7 | cross beam | 8 | secondary positioning lock |
| 9 | main accommodating groove | 10 | X-direction guide rail |
| 11 | main X-direction sliding block | 12 | X-direction motion motor |
| 13 | connecting sleeve | 14 | lower connecting rod |
| 15 | upper connecting rod | 16 | Y-direction drive motor |
| 17 | rotating shaft | 18 | Z-direction drive motor |
| 19 | quick-change mechanism | 20 | lock nut |
| 21 | positioning boss | 22 | groove |
| 23 | connecting buckle | 24 | positioning sleeve |
| 25 | main push rod push rod | 26 | secondary |
| 27 | quick-change mechanism | 28 | laser ranging sensor |
| 29 | fixing plate | 30 | level gauge |
| 31 | main coordinate acquisition needle | 32 | secondary coordinate acquisition needle |
| 33 | main fixing rod | 34 | secondary fixing rod |
| 35 | accommodating cannula | 36 | needle body |
| 37 | shaft-through structure | 38 | rotating drum instrument library and rotary drive mechanism |
| 39 | rotating drum | 40 | rotating table |
| 41 | orthopedic power lifting mechanism | 42 | soft tissue cutting tool lifting mechanism |
| 43 | forceps lifting mechanism | 44 | motor chamber |
| 45 | transmission chamber | 46 | first motor |
| 47 | first guide rail | 48 | first sliding block |
| 49 | orthopedic power connector | 50 | second motor |
| 51 | second guide rail | 52 | second sliding block |
| 53 | cutting tool connector | 54 | third motor |
| 55 | third guide rail | 56 | third sliding block |
| 57 | forceps connector | | |

### DETAILED DESCRIPTION OF THE EMBODIMENTS

Embodiments of the present invention are described in detail below, and it should be understood that the scope of protection of the present invention is not limited by the embodiments and is defined by the appended claims.

Unless there is other explicit expression, throughout the entire description and claims, the term "include/comprise" or its transformations such as "contain" or "including/comprising" or the like will be understood as including the stated elements or components without excluding other elements or other components.

### Embodiment 1

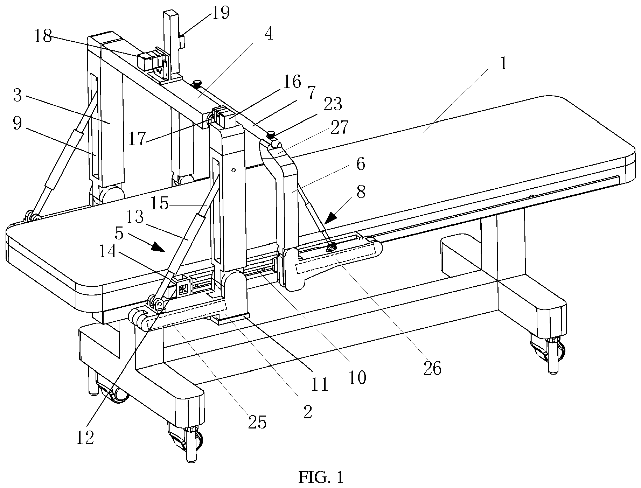

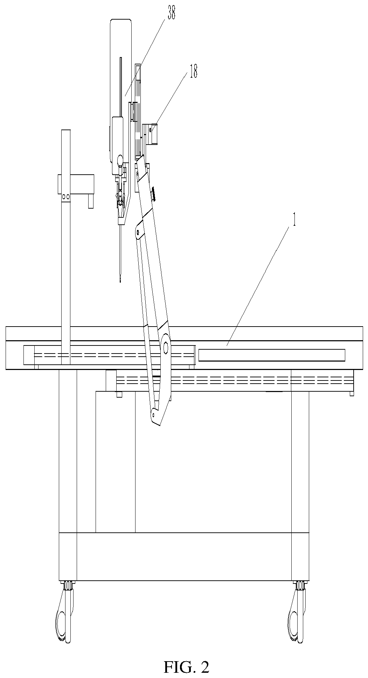

As shown in FIGS. 1 and 2, a detachable and collapsible double-gantry mechanical arm surgical robot includes the operating table 1. X-direction guidance control devices are arranged in pairs on both sides of the operating table 1, in order to implement X-direction motion adjustment of surgical instruments that are subsequently installed. Meanwhile, the main swing arm 3 is installed on the X-direction guidance control device through the main connecting base 2, and the main push rod 25 is installed on the main connecting base 2. The main push rod 25 is connected to the lower end of the main swing arm 3, and pushes the main swing arm 3 to swing. Moreover, the main connecting base 2 and the upper end of the main swing arm 3 are connected to the main positioning lock 5, and the Y-direction guidance device 4 is connected to a Z-direction guidance control device, thereby forming a main gantry mechanical arm. During actual assembly, the rotating shaft 17 is installed at the top of the main swing arm 3, and the main swing arm 3 is respectively provided with a first shaft for connecting the main connecting base 2 and a second shaft for connecting the main push rod 25 (not shown in the figures). Also, in consideration of the execution of multi-posture and multi-instrument surgery, the secondary swing arms 6 are installed on the X-direction guidance control devices through a secondary connecting base, and the cross beam 7 is connected between the secondary swing arms 6, thereby forming a secondary gantry mechanical arm. The secondary push rod 26 is installed on the secondary connecting base, and the secondary push rod 26 is connected to the lower end of the secondary swing arm 6, so as to provide proper support and adjust a height of the cross beam 7. The secondary positioning lock 8 is arranged between the secondary swing arm 6 and the secondary connecting base to increase rigidity of the secondary swing arm 6. In consideration of facilitating the adjustment of a posture of the secondary swing arm 6 and the subsequent switching of instruments, the quick-change mechanism 27 is arranged on the cross beam 7. In conjunction with a preferred embodiment of the present invention, in order to implement ultimate angle adjustment or greatly folding in a non-use state, the main push rod 25 is configured to push the main swing arm 3 to rotate, and the main swing arm 3 is provided with the main accommodating groove 9 for accommodating the main positioning lock 5. Meanwhile, the secondary swing arm 6 is provided with a secondary accommodating groove (not shown in the figures) for accommodating the secondary positioning lock 8. In this way, during transportation or standby maintenance, one end of each of the main positioning lock 5 and the secondary positioning lock 8 can be detached and separated, so as to better enable the main positioning lock 5 and the secondary positioning lock 8 to be correspondingly accommodated in the main accommodating groove 9 and the secondary accommodating groove.

Further, the X-direction motion guidance is considered to satisfy the independent control of the main gantry mechanical arm and the secondary gantry mechanical arm. The X-direction guidance control device includes the X-direction guide rail 10. The main X-direction sliding block 11 and a secondary X-direction sliding block (not shown in the figures) are arranged on the X-direction guide rail 10. The main X-direction sliding block 11 is connected to the main connecting base 2, and the secondary X-direction sliding block is connected to the secondary connecting base. The X-direction motion motor 12 is arranged at one end of the X-direction guide rail 10.

In view of practical implementation, in order to realize the smooth adjustment of the main positioning lock 5 and the secondary positioning lock 8 to achieve better support effect while providing effective motion guidance, each of the main positioning lock 5 and the secondary positioning lock 8 includes the connecting sleeve 13. The upper connecting rod 15 is inserted in the upper end of the connecting sleeve 13, and the lower connecting rod 14 is inserted in the lower end of the connecting sleeve 13. The upper end of the interior of the connecting sleeve 13 is provided with a main right-hand thread, and the lower end of the interior of the connecting sleeve 13 is provided with a main left-hand thread. The portion of the upper connecting rod 15 inserted in the connecting sleeve 13 is provided with a main right-hand thread, and the portion of the lower connecting rod 14 inserted in the connecting sleeve 13 is provided with a main left-hand thread. A nut with forward and reverse rotations is arranged in the connecting sleeve 13. In this way, the adjustment can be realized by rotating the connecting sleeve 13.

Further, in order to satisfy the daily Y-direction adjustment, the Y-direction guidance device 4 includes the Y-direction drive motor 16, and the rotating shaft 17 is installed on the Y-direction drive motor 16. Meanwhile, in order to cooperate with the subsequently installed instruments to implement the Z-axis adjustment and better fit the wounded part for execution, the Z-direction guidance control device includes the Z-direction drive motor 18, and the quick-change mechanism 19 is installed on the Z-direction drive motor 18. In this way, various kinds of instruments are switched for use to match practical requirements of different surgeries.

In order to change the closed gantry structure to an open state, a sterile jacket is installed, and the top end of the main swing arm 3 is connected to the Y-direction guidance device 4 through an opening and closing device. Specifically, the opening and closing device adopted by the present invention includes the positioning sleeve 24. The positioning sleeve 24 is provided with a mounting hole, and the lock nut 20 is arranged in the mounting hole in a penetrating manner. The positioning sleeve 24 is installed at the top of the main swing arm 3 through a lock bolt. Meanwhile, the positioning boss 21 is arranged on the positioning sleeve 24, the groove 22 is arranged at the corresponding position of the top of the main swing arm 3, and the positioning boss 21 is embedded in the groove 22.

Meanwhile, in order to facilitate the disassembly of the cross beam 7 and the combined installation of the subsequent instruments while achieving the installation of the sterile jacket, the connecting buckle 23 is arranged at the top of the secondary swing arm 6, and the cross beam 7 performs positioning and connecting through the connecting buckle 23. The quick-change mechanism 27 is installed on the cross beam 7 to switch various kinds of instruments for use.

It can be seen, through the above literal expression in conjunction with the drawings, that the present invention has the following advantages.
1. The main gantry mechanical arm and the secondary gantry mechanical arm are independent from each other, and instruments are quickly switched through the quick-change structures installed on the Z-direction drive motor and the cross beam, which can implement coordination of multiple surgical instruments and different surgical operations. Moreover, the double-arm configuration is adopted, which enable the double-gantry mechanical arm to have the optimal bearing capacity and the flexible adjustment range. The two arms cooperate with each other to achieve high-precision synchronous operation similar to the operation performed by two hands, which overcomes various shortcomings of the prior single-arm operation, so as to satisfy different operation requirements of a variety of surgeries, have good scalability and adapt to surgical requirements of multiple disciplines.
2. Independent X-direction, Y-direction and Z-direction guidance control devices are arranged, which can cooperate with subsequent control software, so as to control and adjust the multi-axis motion, and improve the accuracy of the surgical operation of the robot.
3. The main gantry mechanical arm and the secondary gantry mechanical arm both can be separately disassembled and assembled, which facilitates the installation of the sterile jacket and ensures the safety and sterility of implementation.
4. The independent push rod structure is adopted, which can provide effective support and height adjustment for each gantry mechanical arm, so as to satisfy the requirements of various kinds of surgeries. After disassembly, the volume is significantly reduced to avoid excessive occupation of the space in the operating room.
5. The overall configuration is simple, which can be combined with various kinds of surgical instruments for use.

### Embodiment 2

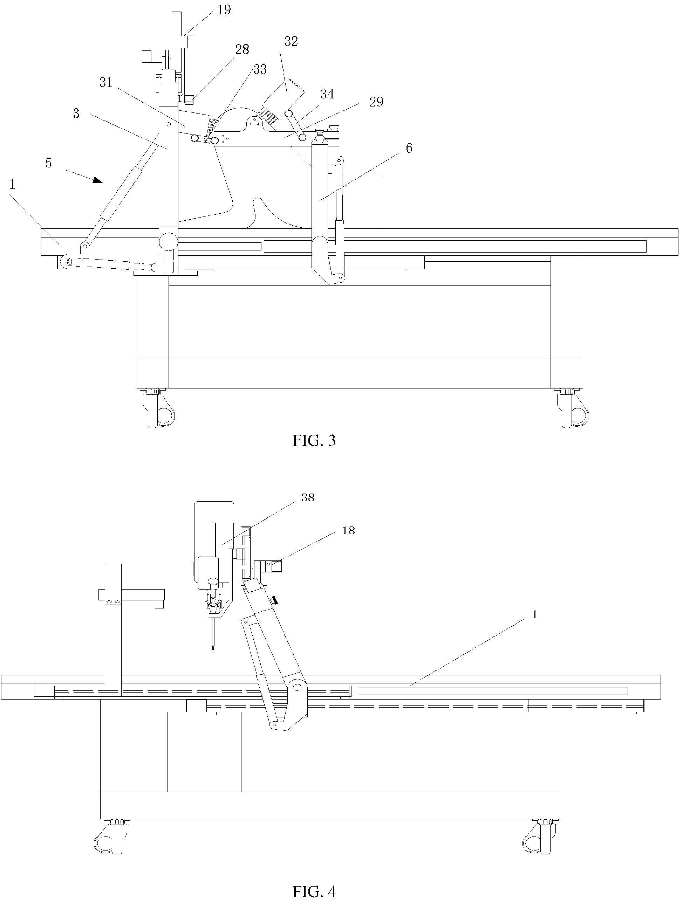

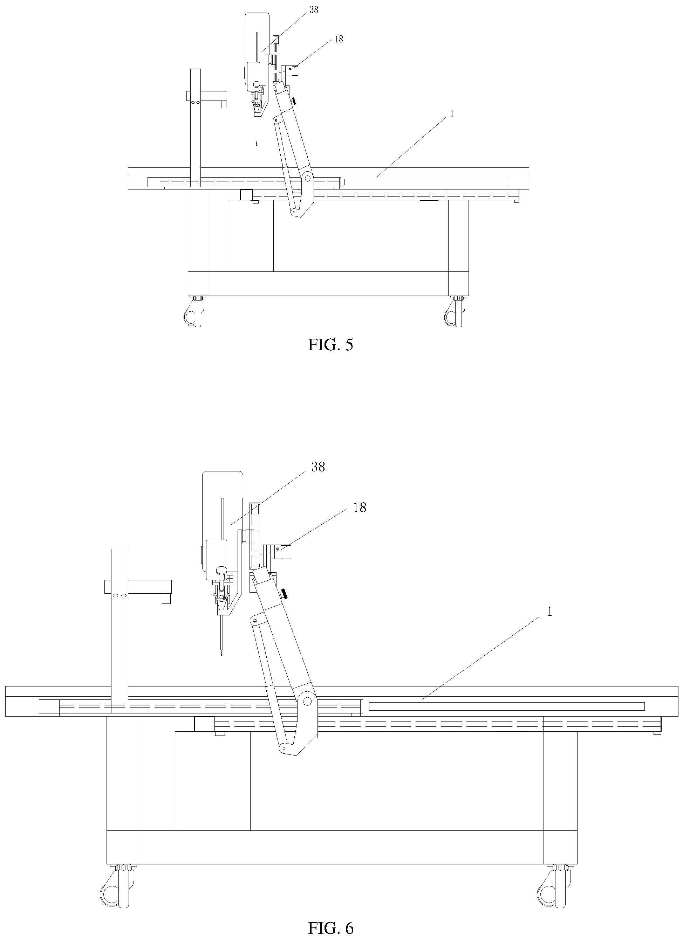

As shown in FIGS. 1 to 5, a double-gantry surgical robot of a bone surface coordinate mapping and acquisition for joint replacement includes the operating table 1. X-direction guidance control devices are arranged in pairs on both sides of the operating table 1, in order to implement X-direction motion adjustment of surgical instruments that are subsequently installed. Meanwhile, the main swing arm 3 is installed on the X-direction guidance control device through the main connecting base 2, and the main push rod 25 is installed on the main connecting base 2. The main push rod 25 is connected to the lower end of the main swing arm 3, and pushes the main swing arm 3 to swing. Moreover, the main connecting base 2 and the upper end of the main swing arm 3 are connected to the main positioning lock 5, and the Y-direction guidance device 4 is connected to a Z-direction guidance control device, thereby forming a main gantry mechanical arm. During actual assembly, the rotating shaft 17 is installed at the top of the main swing arm 3, and the main swing arm 3 is respectively provided with a first shaft for connecting the main connecting base 2 and a second shaft for connecting the main push rod 25 (not shown in the figures). Also, in consideration of the execution of multi-posture and multi-instrument surgery, the secondary swing arms 6 are installed on the X-direction guidance control devices through a secondary connecting base, and the cross beam 7 is connected between the secondary swing arms 6, thereby forming a secondary gantry mechanical arm. The secondary push rod 26 is installed on the secondary connecting base, and the secondary push rod 26 is connected to the lower end of the secondary swing arm 6, so as to provide proper support and adjust a height of the cross beam 7. The secondary positioning lock 8 is arranged between the secondary swing arm 6 and the secondary connecting base to increase rigidity of the secondary swing arm 6. In consideration of facilitating the adjustment of a posture of the secondary swing arm 6 and the subsequent switching of instruments, the quick-change mechanism 27 is arranged on the cross beam 7.

Moreover, in order to acquire subsequent positions of acquisition needles in real time during use, the bone surface coordinates are mapped to the outside of the body through needle tails, and the laser ranging sensor 28 is installed on the Z-direction guidance control device. In view of practical implementation, the laser ranging sensor 28 can be replaced with other devices for sensing the positions of the acquisition needles to satisfy requirements of different implementations, which will not be repeated here. In consideration of better positioning of the j oint to easily acquire the correct bone surface coordinates, in the present invention, a joint fixing device is installed on the cross beam. The joint fixing device includes the fixing plate 29. Specifically, the level gauge 30 for sensing the corresponding position of the joint is installed on the fixing plate 29. In order to acquire the three-dimensional bone surface coordinates, the fixing plate 29 is simultaneously connected to the main coordinate acquisition needle 31 and the secondary coordinate acquisition needle 32.

Moreover, in order to adapt to a naturally retracted arc of a normal j oint, the fixing plate 29 is in a U shape. In consideration of the installation and positioning needs of various accessories, the fixing plate 29 is provided with a fixing hole, and a fixing nail is connected in the fixing hole. In view of actual use, the main fixing rod 33 and the secondary fixing rod 34 are arranged on the fixing plate 29, respectively. The main coordinate acquisition needle 31 is installed on the main fixing rod 33, and the secondary coordinate acquisition needle 32 is installed on the secondary fixing rod 34. In addition, in order to realize array-type data acquisition, each of the main coordinate acquisition needle 31 and the secondary coordinate acquisition needle 32 includes the accommodating cannula 35, and a plurality of needle bodies 36 are distributed in array in the accommodating cannula 35. Moreover, the shaft-through structure 37 is arranged at a side of the accommodating cannula 35, so as to implement shaft-through connection with the main fixing rod 33 or the secondary fixing rod 34.

In conjunction with a preferred embodiment of the present invention, in order to implement ultimate angle adjustment or greatly folding in a non-use state, the main push rod 25 is configured to push the main swing arm 3 to rotate, and the main swing arm 3 is provided with the main accommodating groove 9 for accommodating the main positioning lock 5. Meanwhile, the secondary swing arm 6 is provided with a secondary accommodating groove (not shown in the figures) for accommodating the secondary positioning lock 8. In this way, during transportation or standby maintenance, one end of each of the main positioning lock 5 and the secondary positioning lock 8 can be detached and separated, so as to better enable the main positioning lock 5 and the secondary positioning lock 8 to be correspondingly accommodated in the main accommodating groove 9 and the secondary accommodating groove.

Further, the X-direction motion guidance is considered to satisfy the independent control of the main gantry mechanical arm and the secondary gantry mechanical arm. The X-direction guidance control device includes the X-direction guide rail 10. The main X-direction sliding block 11 and a secondary X-direction sliding block (not shown in the figures) are arranged on the X-direction guide rail 10. The main X-direction sliding block 11 is connected to the main connecting base 2, and the secondary X-direction sliding block is connected to the secondary connecting base. The X-direction motion motor 12 is arranged at one end of the X-direction guide rail 10.

In view of practical implementation, in order to realize the smooth adjustment of the main positioning lock 5 and the secondary positioning lock 8 to achieve better support effect while providing effective motion guidance, each of the main positioning lock 5 and the secondary positioning lock 8 includes the connecting sleeve 13. The upper connecting rod 15 is inserted in the upper end of the connecting sleeve 13, and the lower connecting rod 14 is inserted in the lower end of the connecting sleeve 13. The upper end of the interior of the connecting sleeve 13 is provided with a main right-hand thread, and the lower end of the interior of the connecting sleeve 13 is provided with a main left-hand thread. The portion of the upper connecting rod 15 inserted in the connecting sleeve 13 is provided with a main right-hand thread, and the portion of the lower connecting rod 14 inserted in the connecting sleeve 13 is provided with a main left-hand thread. A nut with forward and reverse rotations is arranged in the connecting sleeve 13. In this way, the adjustment can be realized by rotating the connecting sleeve 13. Of course, the main positioning lock 5 and the secondary positioning lock 8 can be replaced with any mechanism capable of locking and supporting the positions of the main gantry mechanical arm and the secondary gantry mechanical arm, as long as the positioning use and safety of each gantry mechanical arm can be satisfied.

Further, in order to satisfy the daily Y-direction adjustment, the Y-direction guidance device 4 includes the Y-direction drive motor 16, and the rotating shaft 17 is installed on the Y-direction drive motor 16. Meanwhile, in order to cooperate with the subsequently installed instruments to implement the Z-axis adjustment and better fit the wounded part for execution, the Z-direction guidance control device includes the Z-direction drive motor 18, and the quick-change mechanism 19 is installed on the Z-direction drive motor 18. In this way, various kinds of cutting instruments are switched for use to match practical requirements of different joint replacement surgeries.

In order to change the closed gantry structure to an open state, a sterile jacket is installed, and the top end of the main swing arm 3 is connected to the Y-direction guidance device 4 through an opening and closing device. Specifically, the opening and closing device adopted by the present invention includes the positioning sleeve 24. The positioning sleeve 24 is provided with a mounting hole, and the lock nut 20 is arranged in the mounting hole in a penetrating manner. The positioning sleeve 24 is installed at the top of the main swing arm 3 through a lock bolt. Meanwhile, the positioning boss 21 is arranged on the positioning sleeve 24, the groove 22 is arranged at the corresponding position of the top of the main swing arm 3, and the positioning boss 21 is embedded in the groove 22.

Meanwhile, in order to facilitate the disassembly of the cross beam 7 and the combined installation of the subsequent instruments while achieving the installation of the sterile jacket, the connecting buckle 23 is arranged at the top of the secondary swing arm 6, and the cross beam 7 performs positioning and connecting through the connecting buckle 23. The quick-change mechanism 27 is installed on the cross beam 7 to switch various kinds of instruments for use.

The working principle of the present invention is as follows:
Taking the knee arthroplasty as an example, the main positioning lock extends or contracts to push the main swing arm to ascend or descend, so as to adapt to different patients. When the main swing arm contracts to the shortest, the main swing arm can be retracted to flush with the surface of the operating table. Since the combination of the main positioning lock and the main swing arm has good stability and almost does not have a cantilever, the robot has good rigidity.

During use, since the main coordinate acquisition needles and the secondary coordinate acquisition needles are in a cannula-type matrix-shaped structure, the needle tips can pierce the periosteum, and the bone surface coordinates can be mapped to the outside of the body through the needle tails. In this way, the influence of periosteum coverage, bleeding, body fluid and others on the acquisition of the bone coordinate points during the joint replacement surgery can be ideally avoided.

Meanwhile, the laser ranging sensor scans a plurality of points of the needle tails as the main gantry mechanical arm moves, so that after being acquired, the coordinates of the bone surface at the surgically exposed position can be registered to determine the line of gravity of lower limb to perform osteotomy operation.

It can be seen, through the above literal expression in conjunction with the drawings, that the present invention has the following advantages.
1. The main gantry mechanical arm and the secondary gantry mechanical arm are independent from each other. The main gantry mechanical arm and the secondary gantry mechanical arm have flexible adjustment ranges and have a good function of fixing bones (lower limbs such as femur and tibia). The main gantry mechanical arm has the optimal bearing capacity, and quickly switches the instruments through the quick-change structures installed on the Z-direction drive motor and the cross beam. Through the mutual cooperation of the configuration of the two arms, where one firmly grasps and the other flexibly cuts, the high-precision synchronous operation similar to the operation performed by two hands can be achieved, which greatly improves the accuracy of joint surgery.
2. Independent X-direction, Y-direction and Z-direction guidance control devices are arranged, which can cooperate with subsequent control software, so as to control and adjust the multi-axis motion, improve the accuracy of the surgical operation of the joint replacement robot and expand the scope of application.
3. The main gantry mechanical arm and the secondary gantry mechanical arm both can be separately disassembled and assembled, which facilitates the installation of the sterile jacket and ensures the safety and sterility of implementation.
4. The main coordinate acquisition needle, the secondary coordinate acquisition needle and the laser ranging sensor cooperate with each other to map the bone surface coordinates to the outside of the body, which facilitates providing an accurate position reference, so that the influence of periosteum coverage, bleeding, body fluid and others on the acquisition of the bone coordinate points during the joint replacement surgery can be ideally avoided.

### Embodiment 3

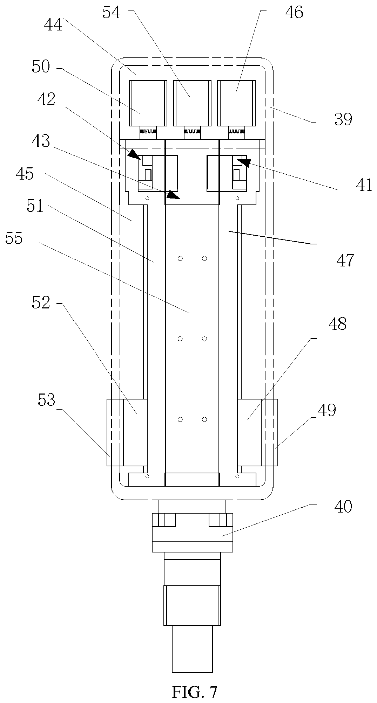

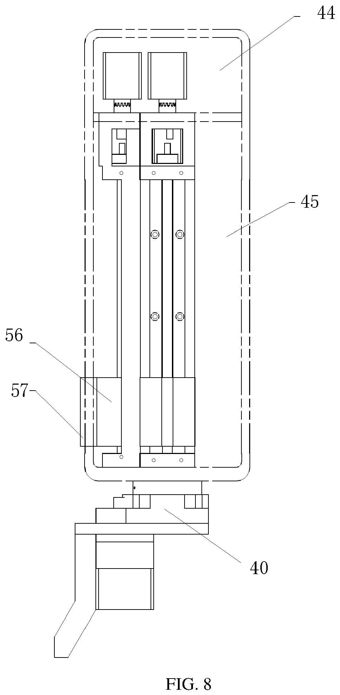

As shown in FIGS. 1, 2, 6, 7 and 8, a gantry collapsible mechanical arm spinal surgical robot includes the operating table 1. X-direction guidance control devices are arranged in pairs on both sides of the operating table 1, in order to implement X-direction motion adjustment of surgical instruments that are subsequently installed. Meanwhile, the main swing arm 3 is installed on the X-direction guidance control device through the main connecting base 2, and the main push rod 25 is installed on the main connecting base 2. The main push rod 25 is connected to the lower end of the main swing arm 3, and pushes the main swing arm 3 to swing. Moreover, the main connecting base 2 and the upper end of the main swing arm 3 are connected to the main positioning lock 5, and the Y-direction guidance device 4 is connected to a Z-direction guidance control device, thereby forming a main gantry mechanical arm. During actual assembly, the rotating shaft 17 is installed at the top of the main swing arm 3, and the main swing arm 3 is respectively provided with a first shaft for connecting the main connecting base 2 and a second shaft for connecting the main push rod 25 (not shown in the figures). Also, in consideration of the execution of multi-posture and multi-instrument surgery, the secondary swing arms 6 are installed on the X-direction guidance control devices through a secondary connecting base, and the cross beam 7 is connected between the secondary swing arms 6, thereby forming a secondary gantry mechanical arm. The secondary push rod 26 is installed on the secondary connecting base, and the secondary push rod 26 is connected to the lower end of the secondary swing arm 6, so as to provide proper support and adjust a height of the cross beam 7. The secondary positioning lock 8 is arranged between the secondary swing arm 6 and the secondary connecting base to increase rigidity of the secondary swing arm 6. In consideration of facilitating the adjustment of a posture of the secondary swing arm 6 and the subsequent switching of instruments, the quick-change mechanism 27 is arranged on the cross beam 7. The rotating drum instrument library and rotary drive mechanism 38 is installed on the secondary gantry mechanical arm, and includes the rotating drum 39. The rotating table 40 is arranged at the bottom of the rotating drum 39. The orthopedic power lifting mechanism 41, the soft tissue cutting tool lifting mechanism 42 and the forceps lifting mechanism 43 are respectively arranged in the rotating drum 39. The upper end of the rotating drum 39 forms the motor chamber 44, and the lower end of the rotating drum 39 forms the transmission chamber 45.

In conjunction with a preferred embodiment of the present invention, in order to implement effective stroke control on various kinds of orthopedic surgical instruments that are subsequently installed, the adopted orthopedic power lifting mechanism 41 includes the first motor 46 located in the motor chamber 44. The lower end of the first motor 46 is connected to the first guide rail 47 located in the transmission chamber 45. The first sliding block 48 is installed on the first guide rail 47, and the orthopedic power connector 49 is installed on the first sliding block 48. Meanwhile, in order to cooperate with the work of the soft tissue cutting tool, the adopted soft tissue cutting tool lifting mechanism 42 includes the second motor 50 located in the motor chamber 44. The lower end of the second motor 50 is connected to the second guide rail 51 located in the transmission chamber 45. The second sliding block 52 is installed on the second guide rail 51, and the soft tissue cutting tool connector 53 is installed on the second sliding block 52. Moreover, for the accurate running of the forceps devices during the surgery implementation, the adopted forceps lifting mechanism 43 includes the third motor 54 located in the motor chamber 44. The lower end of the third motor 54 is connected to the third guide rail 55 located in the transmission chamber 45. The third sliding block 56 is installed on the third guide rail 55, and the forceps connector 57 is installed on the third sliding block 56.

Further, the X-direction motion guidance is considered to satisfy the independent control of the main gantry mechanical arm and the secondary gantry mechanical arm. The X-direction guidance control device includes the X-direction guide rail 10. The main X-direction sliding block 11 and a secondary X-direction sliding block (not shown in the figures) are arranged on the X-direction guide rail 10. The main X-direction sliding block 11 is connected to the main connecting base 2, and the secondary X-direction sliding block is connected to the secondary connecting base. The X-direction motion motor 12 is arranged at one end of the X-direction guide rail 10.

Meanwhile, in order to satisfy the daily Y-direction adjustment, the Y-direction guidance device 4 includes the Y-direction drive motor 16, and the rotating shaft 17 is installed on the Y-direction drive motor 16. Meanwhile, in order to cooperate with the subsequently installed instruments to implement the Z-axis adjustment and better fit the wounded part for execution, the Z-direction guidance control device includes the Z-direction drive motor 18, and the quick-change mechanism 19 is installed on the Z-direction drive motor 18. In this way, various kinds of instruments are switched for use to match practical requirements of different surgeries.

In order to change the closed gantry structure to an open state, a sterile jacket is installed, and the top end of the main swing arm 3 is connected to the Y-direction guidance device 4 through an opening and closing device. Specifically, the opening and closing device adopted by the present invention includes the positioning sleeve 24. The positioning sleeve 24 is provided with a mounting hole, and the lock nut 20 is arranged in the mounting hole in a penetrating manner. The positioning sleeve 24 is installed at the top of the main swing arm 3 through a lock bolt. Meanwhile, the positioning boss 21 is arranged on the positioning sleeve 24, the groove 22 is arranged at the corresponding position of the top of the main swing arm 3, and the positioning boss 21 is embedded in the groove 22.

Meanwhile, in order to facilitate the disassembly of the cross beam 7 and the combined installation of the subsequent instruments while achieving the installation of the sterile jacket, the connecting buckle 23 is arranged at the top of the secondary swing arm 6, and the cross beam 7 performs positioning and connecting through the connecting buckle 23. The quick-change mechanism 27 is installed on the cross beam 7 to switch various kinds of instruments for use.

In view of practical implementation, in order to realize the smooth adjustment of the main positioning lock 5 and the secondary positioning lock 8 to achieve better support effect while providing effective motion guidance, each of the main positioning lock 5 and the secondary positioning lock 8 includes the connecting sleeve 13. The upper connecting rod 15 is inserted in the upper end of the connecting sleeve 13, and the lower connecting rod 14 is inserted in the lower end of the connecting sleeve 13. The upper end of the interior of the connecting sleeve 13 is provided with a main right-hand thread, and the lower end of the interior of the connecting sleeve 13 is provided with a main left-hand thread. The portion of the upper connecting rod 15 inserted in the connecting sleeve 13 is provided with a main right-hand thread, and the portion of the lower connecting rod 14 inserted in the connecting sleeve 13 is provided with a main left-hand thread. A nut with forward and reverse rotations is arranged in the connecting sleeve 13. In this way, the adjustment can be realized by rotating the connecting sleeve 13.

It can be seen, through the above literal expression in conjunction with the drawings, that the present invention has the following advantages.
1. The main gantry mechanical arm and the secondary gantry mechanical arm are independent from each other, and the instruments are quickly and automatically switched through a quick-change structure of the rotating drum instrument library, so as to implement the high-precision coordinated operation similar to the operation performed by two hands and even multiple hands.
2. The present invention has large bearing capacity and good rigidity. Since the gantry structure has the relatively strong rigidity and a very short cantilever, various shortcomings of the prior single-arm j oint-type robots are overcome to install a variety of surgical instruments while implementing heavy load, so as to satisfy different operation requirements of various kinds of spinal surgeries.
3. Independent X-direction, Y-direction and Z-direction guidance control devices are arranged, which can cooperate with subsequent control software, so as to implement multi-axis motion control and adjustment and implement separately independent control of orthopedic instruments, forceps instruments and soft tissue cutting tools, thereby improving the flexibility and accuracy of the surgical operation of the robot.
4. The main gantry mechanical arm and the secondary gantry mechanical arm both can be separately disassembled and assembled, which facilitates the installation of the sterile jacket and ensures the safety and sterility of implementation.
5. The independent push rod structure is adopted, which can provide effective support and height adjustment for each gantry mechanical arm, so as to satisfy the requirements of various kinds of surgeries. After disassembly, the volume is significantly reduced to avoid excessive occupation of the space in the operating room.
6. The rotating drum instrument library has good scalability, which can satisfy requirements of various kinds of spinal surgeries and even requirements of surgeries of other disciplines.

The aforementioned specific exemplary embodiments of the present invention is for the purpose of description and illustration. The depiction does not intend to limit the present invention as the disclosed accurate form, and it is obvious that many changes and modifications may be made according to the above statements, while staying within the scope of the appended claims. The purpose of selecting and describing the exemplary embodiments lies in explaining the specific principle of the present invention and its practical application, so that those skilled in the art can implement and utilize a variety of different exemplary implementation solutions and a variety of different selections and changes of the present invention. The scope of the present invention is defined by the claims.

## Claims

1. A detachable and collapsible double-gantry mechanical arm surgical robot, comprising an operating table (1) having a longitudinal dimension, a transversal dimension and a vertical dimension, wherein X-direction guidance control devices are arranged in pairs on both longitudinal sides of the operating table (1); main swing arms (3) are connected to the operating table (1) through a main connecting base (2); a main push rod (25) is installed on the main connecting base (2); the main push rod (25) is connected to a lower end of the main swing arm (3); the main connecting base (2) and an upper end of the main swing arm (3) are connected to a main positioning lock (5); the main swing arm (3) is further connected to a Y-direction guidance device (4); and the Y-direction guidance device (4) is connected to a Z-direction guidance control device, wherein a main gantry mechanical arm is formed, main positioning lock (5) locks and supports the main gantry mechanical arm;
secondary swing arms (6) are connected to the operating table (1) through a secondary connecting base installed on the X-direction guidance control devices, and a cross beam (7) is connected between the secondary swing arms (6) through a quick-change mechanism (27), wherein a secondary gantry mechanical arm is formed; and
a secondary push rod (26) is connected between the secondary connecting base and the secondary swing arm (6); the secondary push rod (26) is connected to a lower end of the secondary swing arm (6); and a secondary positioning lock (8) for increasing rigidity of the secondary swing arm (6) is arranged between the secondary swing arm (6) and the secondary connecting base such that the secondary positioning lock (8) locks and supports the secondary gantry mechanical arm; wherein the longitudinal dimension of the operating table (1) is defined as X-direction; the transversal dimension of the operating table (1) is defined as Y-direction and, the vertical dimension of the operating table (1) is defined as Z-direction; X, Y, Z reference frame formed by the X-direction, Y-direction, Z-direction is orthogonal; **and characterized in that**
the main connecting base (2) is installed on the X-direction guidance control devices;
the main push rod (25) pushes the main swing arm (3) to rotate around a main swing axis parallel to the Y-direction;
the secondary push rod (26) pushes the secondary swing arm (6) to rotate around a secondary swing axis parallel to the Y-direction.

2. The detachable and collapsible double-gantry mechanical arm surgical robot of claim 1, wherein the X-direction guidance control device comprises an X-direction guide rail (10); a main X-direction sliding block (11) and a secondary X-direction sliding block are arranged on the X-direction guide rail (10); the main X-direction sliding block (11) is connected to the main connecting base (2); the secondary X-direction sliding block is connected to the secondary connecting base; and an X-direction motion motor (12) is arranged at one end of the X-direction guide rail (10).

3. The detachable and collapsible double-gantry mechanical arm surgical robot of claim 1, wherein a top end of the main swing arm (3) is connected to the Y-direction guidance device (4) through an opening and closing device; the opening and closing device comprises a positioning sleeve (24); the positioning sleeve (24) is provided with a mounting hole, and a lock nut (20) is arranged in the mounting hole in a penetrating manner; the positioning sleeve (24) is installed at a top of the main swing arm (3) through a lock bolt; a positioning boss (21) is arranged on the positioning sleeve (24), a groove (22) is arranged at a corresponding position of the top of the main swing arm (3), and the positioning boss (21) is embedded in the groove (22).

4. The detachable and collapsible double-gantry mechanical arm surgical robot of claim 1 or 3, wherein the main swing arm (3) is provided with a main accommodating groove (9) for accommodating the main positioning lock (5), and the secondary swing arm (6) is provided with a secondary accommodating groove for accommodating the secondary positioning lock (8).

5. The detachable and collapsible double-gantry mechanical arm surgical robot of claim 4, wherein each of the main positioning lock (5) and the secondary positioning lock (8) comprises a connecting sleeve (13); an upper connecting rod (15) is inserted in an upper end of the connecting sleeve (13), and a lower connecting rod (14) is inserted in a lower end of the connecting sleeve (13); an upper end of an interior of the connecting sleeve (13) is provided with a main right-hand thread, and a lower end of the interior of the connecting sleeve (13) is provided with a main left-hand thread; a portion of the upper connecting rod (15) inserted in the connecting sleeve (13) is provided with a main right-hand thread, and a portion of the lower connecting rod (14) inserted in the connecting sleeve (13) is provided with a main left-hand thread; and a nut with forward and reverse rotations is arranged in the connecting sleeve (13).

6. The detachable and collapsible double-gantry mechanical arm surgical robot of claim 4, wherein a connecting buckle (23) is arranged at a top of the secondary swing arm (6), and the cross beam (7) performs positioning and connecting through the connecting buckle (23).

7. The detachable and collapsible double-gantry mechanical arm surgical robot of claim 1 or 3, wherein the Y-direction guidance device (4) comprises a Y-direction drive motor (16); a rotating shaft (17) is installed on the Y-direction drive motor (16); and the main push rod (25) is connected between the main connecting base (2) and the main swing arm (3).

8. The detachable and collapsible double-gantry mechanical arm surgical robot of claim 1, wherein a laser ranging sensor (28) is installed on the Z-direction guidance control device; a joint fixing device is installed on the cross beam (7); the joint fixing device comprises a fixing plate (29); a level gauge (30) is installed on the fixing plate (29); and the fixing plate (29) is simultaneously connected to a main coordinate acquisition needle (31) and a secondary coordinate acquisition needle (32).

9. The detachable and collapsible double-gantry mechanical arm surgical robot of claim 8, wherein the fixing plate (29) is in a U shape; the fixing plate (29) is provided with a fixing hole, and a fixing nail is connected in the fixing hole; a main fixing rod (33) and a secondary fixing rod (34) are arranged on the fixing plate (29), respectively; the main coordinate acquisition needle (31) is installed on the main fixing rod (33), and the secondary coordinate acquisition needle (32) is connected on the secondary fixing rod (34).

10. The detachable and collapsible double-gantry mechanical arm surgical robot of claim 8, wherein each of the main coordinate acquisition needle (31) and the secondary coordinate acquisition needle (32) comprises an accommodating cannula (35); a plurality of needle bodies (36) are distributed in array in the accommodating cannula (35); and a shaft-through structure (37) is arranged at a side of the accommodating cannula (35).

11. The detachable and collapsible double-gantry mechanical arm surgical robot of claim 1 or 8, wherein the Z-direction guidance control device comprises a Z-direction drive motor (18), and a quick-change mechanism (19) is installed on the Z-direction drive motor (18).

12. The detachable and collapsible double-gantry mechanical arm surgical robot of claim 1, wherein a rotating drum instrument library and rotary drive mechanism (38) is installed on the secondary gantry mechanical arm, and comprises a rotating drum (39); a rotating table (40) is arranged at a bottom of the rotating drum (39); an orthopedic power lifting mechanism (41), a soft tissue cutting tool lifting mechanism (42) and a forceps lifting mechanism (43) are arranged in the rotating drum (39), respectively; an upper end of the rotating drum (39) forms a motor chamber (44), and a lower end of the rotating drum (39) forms a transmission chamber (45).

13. The detachable and collapsible double-gantry mechanical arm surgical robot of claim 12, wherein the orthopedic power lifting mechanism (41) comprises a first motor (46) located in the motor chamber (44); a lower end of the first motor (46) is connected to a first guide rail (47) located in the transmission chamber (45); a first sliding block (48) is installed on the first guide rail (47), and an orthopedic power connector (49) is installed on the first sliding block (48).

14. The detachable and collapsible double-gantry mechanical arm surgical robot of claim 12, wherein the soft tissue cutting tool lifting mechanism (42) comprises a second motor (50) located in the motor chamber (44); a lower end of the second motor (50) is connected to a second guide rail (51) located in the transmission chamber (45); a second sliding block (52) is installed on the second guide rail (51), and a soft tissue cutting tool connector (53) is installed on the second sliding block (52).

15. The detachable and collapsible double-gantry mechanical arm surgical robot of claim 12, wherein the forceps lifting mechanism (43) comprises a third motor (54) located in the motor chamber (44); a lower end of the third motor (54) is connected to a third guide rail (55) located in the transmission chamber (45); a third sliding block (56) is installed on the third guide rail (55), and a forceps connector (57) is installed on the third sliding block (56).

## Patentansprüche

1. Chirurgischer Roboter mit lösbarem und zusammenklappbarem mechanischem Doppelkranarm, umfassend einen Operationstisch (1) mit einer Längsabmessung, einer Querabmessung und einer vertikalen Abmessung, wobei X-Richtungsführungssteuervorrichtungen paarweise auf beiden Längsseiten des Operationstisches (1) angeordnet sind; Hauptschwenkarme (3) durch einen Hauptverbindungssockel (2) mit dem Operationstisch (1) verbunden sind; eine Hauptschubstange (25) an dem Hauptverbindungssockel (2) installiert ist; die Hauptschubstange (25) mit einem unteren Ende des Hauptschwenkarms (3) verbunden ist; der Hauptverbindungssockel (2) und ein oberes Ende des Hauptschwenkarms (3) mit einer Hauptpositionierungsarretierung (5) verbunden sind; der Hauptschwenkarm (3) ferner mit einer Y-Richtungsführungsvorrichtung (4) verbunden ist; und die Y-Richtungsführungsvorrichtung (4) mit einer Z-Richtungsführungssteuervorrichtung verbunden ist, wodurch ein mechanischer Hauptkranarm gebildet ist, wobei die Hauptpositionierungsarretierung (5) den mechanischen Hauptkranarm arretiert und trägt;
sekundäre Schwenkarme (6) durch einen sekundären Verbindungssockel, der an den X-Richtungsführungssteuervorrichtungen installiert ist, mit dem Operationstisch (1) verbunden sind, und ein Querträger (7) durch einen Schnellwechselmechanismus (27) zwischen den sekundären Schwenkarmen (6) verbunden ist, wodurch ein sekundärer mechanischer Kranarm gebildet ist; und
eine sekundäre Schubstange (26) zwischen dem sekundären Verbindungssockel und dem sekundären Schwenkarm (6) verbunden ist; die sekundäre Schubstange (26) mit einem unteren Ende des sekundären Schwenkarms (6) verbunden ist; und eine sekundäre Positionierungsarretierung (8) zur Erhöhung der Steifigkeit des sekundären Schwenkarms (6) zwischen dem sekundären Schwenkarm (6) und dem sekundären Verbindungssockel angeordnet ist, derart, dass die sekundäre Positionierungsarretierung (8) den sekundären mechanischen Kranarm arretiert und trägt; wobei die Längsabmessung des Operationstisches (1) als X-Richtung definiert ist; die Querabmessung des Operationstisches (1) als Y-Richtung definiert ist und die vertikale Abmessung des Operationstisches (1) als Z-Richtung definiert ist; ein durch die X-Richtung, Y-Richtung, Z-Richtung gebildeter X,Y,Z-Bezugsrahmen orthogonal ist; und **dadurch gekennzeichnet, dass**
der Hauptverbindungssockel (2) an den X-Richtungsführungssteuervorrichtungen installiert ist;
die Hauptschubstange (25) den Hauptschwenkarm (3) um eine Hauptschwenkachse parallel zur Y-Richtung dreht;
die sekundäre Schubstange (26) den sekundären Schwenkarm (6) um eine sekundäre Schwenkachse parallel zur Y-Richtung dreht.

2. Chirurgischer Roboter mit lösbarem und zusammenklappbarem mechanischem Doppelkranarm nach Anspruch 1, wobei die X-Richtungsführungssteuervorrichtung eine X-Richtungsführungsschiene (10) umfasst; ein X-Richtungshauptgleitblock (11) und ein sekundärer X-Richtungsgleitblock an der X-Richtungsführungsschiene (10) angeordnet sind; der X-Richtungshauptgleitblock (11) mit dem Hauptverbindungssockel (2) verbunden ist; der sekundäre X-Richtungsgleitblock mit dem sekundären Verbindungssockel verbunden ist; und ein X-Richtungsbewegungsmotor (12) an einem Ende der X-Richtungsführungsschiene (10) angeordnet ist.

3. Chirurgischer Roboter mit lösbarem und zusammenklappbarem mechanischem Doppelkranarm nach Anspruch 1, wobei ein oberes Ende des Hauptschwenkarms (3) durch eine Öffnungs- und Schließvorrichtung mit der Y-Richtungsführungsvorrichtung (4) verbunden ist; die Öffnungs- und Schließvorrichtung eine Positionierungshülse (24) umfasst; die Positionierungshülse (24) mit einem Montageloch versehen ist und eine Befestigungsmutter (20) durch das Montageloch verlaufend angeordnet ist; die Positionierungshülse (24) durch einen Arretierungsbolzen an einer Oberseite des Hauptschwenkarms (3) installiert ist; ein Positionierungsansatz (21) an der Positionierungshülse (24) angeordnet ist, eine Nut (22) an einer entsprechenden Position der Oberseite des Hauptschwenkarms (3) angeordnet ist und der Positionierungsansatz (21) in die Nut (22) eingebettet ist.

4. Chirurgischer Roboter mit lösbarem und zusammenklappbarem mechanischem Doppelkranarm nach Anspruch 1 oder 3, wobei the Hauptschwenkarm (3) mit einer Hauptaufnahmenut (9) zum Aufnehmen der Hauptpositionierungsarretierung (5) versehen ist und der sekundäre Schwenkarm (6) mit einer sekundären Aufnahmenut zum Aufnehmen der sekundären Positionierungsarretierung (8) versehen ist.

5. Chirurgischer Roboter mit lösbarem und zusammenklappbarem mechanischem Doppelkranarm nach Anspruch 4, wobei die Hauptpositionierungsarretierung (5) und die sekundäre Positionierungsarretierung (8) jeweils eine Verbindungshülse (13) umfassen; eine obere Verbindungsstange (15) in eine oberes Ende der Verbindungshülse (13) eingeführt ist und eine untere Verbindungsstange (14) in ein unteres Ende der Verbindungshülse (13) eingeführt ist; eine oberes Ende eines Inneren der Verbindungshülse (13) mit einem rechtsgängigen Hauptgewinde versehen ist und ein unteres Ende des Inneren der Verbindungshülse (13) mit einem linksgängigen Hauptgewinde versehen ist; ein Abschnitt der oberen Verbindungsstange (15), der in die Verbindungshülse (13) eingeführt ist, mit einem rechtsgängigen Hauptgewinde versehen ist und eine Abschnitt der unteren Verbindungsstange (14), der in die Verbindungshülse (13) eingeführt ist, mit einem linksgängigen Hauptgewinde versehen ist; und eine Mutter mit Vorwärts- und Rückwärtsdrehung in der Verbindungshülse (13) angeordnet ist.

6. Chirurgischer Roboter mit lösbarem und zusammenklappbarem mechanischem Doppelkranarm nach Anspruch 4, wobei eine Verbindungsnase (23) an einer Oberseite des sekundären Schwenkarms (6) angeordnet ist und der Querträger (7) durch die Verbindungsnase (23) eine Positionierung durchführt.

7. Chirurgischer Roboter mit lösbarem und zusammenklappbarem mechanischem Doppelkranarm nach Anspruch 1 oder 3, wobei die Y-Richtungsführungsvorrichtung (4) einen Y-Richtungsantriebsmotor (16) umfasst; eine Drehwelle (17) an dem Y-Richtungsantriebsmotor (16) installiert ist; und die Hauptschubstange (25) zwischen dem Hauptverbindungssockel (2) und dem Hauptschwenkarm (3) verbunden ist.

8. Chirurgischer Roboter mit lösbarem und zusammenklappbarem mechanischem Doppelkranarm nach Anspruch 1, wobei ein Laserentfernungssensor (28) an der Z-Richtungsführungssteuervorrichtung installiert ist; eine Gelenkfixierungsvorrichtung an dem Querträger (7) installiert ist; die Gelenkfixierungsvorrichtung eine Fixierungsplatte (29) umfasst; ein Höhenmesser (30) an der Fixierungsplatte (29) installiert ist; und die Fixierungsplatte (29) gleichzeitig mit einer Hauptkoordinatenerfassungsnadel (31) und einer sekundären Koordinatenerfassungsnadel (32) verbunden ist.

9. Chirurgischer Roboter mit lösbarem und zusammenklappbarem mechanischem Doppelkranarm nach Anspruch 8, wobei die Fixierungsplatte (29) U-förmig ist; die Fixierungsplatte (29) mit einem Fixierungsloch versehen ist und ein Fixierungsnagel in dem Fixierungsloch verbunden ist; eine Hauptfixierungsstange (33) und eine sekundäre Fixierungsstange (34) an der Fixierungsplatte (29) angeordnet sind; die Hauptkoordinatenerfassungsnadel (31) an der Hauptfixierungsstange (33) installiert ist und die sekundäre Koordinatenerfassungsnadel (32) mit der sekundären Fixierungsstange (34) verbunden ist.

10. Chirurgischer Roboter mit lösbarem und zusammenklappbarem mechanischem Doppelkranarm nach Anspruch 8, wobei die Hauptkoordinatenerfassungsnadel (31) und die sekundäre Koordinatenerfassungsnadel (32) eine Aufnahmekanüle (35) umfassen; eine Vielzahl von Nadelkörpern (36) in einer Reihe in der Aufnahmekanüle (35) verteilt ist; und eine Schaftdurchgangsstruktur (37) auf einer Seite der Aufnahmekanüle (35) angeordnet ist.

11. Chirurgischer Roboter mit lösbarem und zusammenklappbarem mechanischem Doppelkranarm nach Anspruch 1 oder 8, wobei die Z-Richtungsführungssteuervorrichtung einen Z-Richtungsantriebsmotor (18) umfasst und ein Schnellwechselmechanismus (19) an dem Z-Richtungsantriebsmotor (18) installiert ist.

12. Chirurgischer Roboter mit lösbarem und zusammenklappbarem mechanischem Doppelkranarm nach Anspruch 1, wobei ein Drehtrommelinstrumentenlager und ein Drehantriebsmechanismus (38) an dem sekundären mechanischen Kranarm installiert sind und eine Drehtrommel (39) umfassen; ein Drehtisch (40) an einer Unterseite der Drehtrommel (39) angeordnet ist; ein orthopädischer Servohebemechanismus (41), ein Weichgewebeschneidwerkzeughebemechanismus (42) und ein Zangenhebemechanismus (43) in der Drehtrommel (39) angeordnet sind; ein oberes Ende der Drehtrommel (39) eine Motorkammer (44) bildet und ein unteres Ende der Drehtrommel (39) eine Getriebekammer (45) bildet.

13. Chirurgischer Roboter mit lösbarem und zusammenklappbarem mechanischem Doppelkranarm nach Anspruch 12, wobei der orthopädische Servohebemechanismus (41) einen ersten Motor (46) umfasst, der sich in der Motorkammer (44) befindet; ein unteres Ende des ersten Motors (46) mit einer ersten Führungsschiene (47) verbunden ist, die sich in der Getriebekammer (45) befindet; eine erster Gleitblock (48) an der ersten Führungsschiene (47) installiert ist und ein orthopädisches Servoverbindungselement (49) an dem ersten Gleitblock (48) installiert ist.

14. Chirurgischer Roboter mit lösbarem und zusammenklappbarem mechanischem Doppelkranarm nach Anspruch 12, wobei der Weichgewebeschneidwerkzeughebemechanismus (42) einen zweiten Motor (50) umfasst, der sich in der Motorkammer (44) befindet; ein unteres Ende des zweiten Motors (50) mit einer zweiten Führungsschiene (51) verbunden ist, die sich in der Getriebekammer (45) befindet; ein zweiter Gleitblock (52) an der zweiten Führungsschiene (51) installiert ist und ein Weichgewebeschneidwerkzeugverbindungselement (53) an dem zweiten Gleitblock (52) installiert ist.

15. Chirurgischer Roboter mit lösbarem und zusammenklappbarem mechanischem Doppelkranarm nach Anspruch 12, wobei der Zangenhebemechanismus (43) einen dritten Motor (54) umfasst, der sich in der Motorkammer (44) befindet; ein unteres Ende des dritten Motors (54) mit einer dritten Führungsschiene (55) verbunden ist, die sich in der Getriebekammer (45) befindet; ein dritter Gleitblock (56) an der dritten Führungsschiene (55) installiert ist und ein Zangenverbindungselement (57) an dem dritten Gleitblock (56) installiert ist.

## Revendications

1. Un robot chirurgical à bras mécanique double-portique détachable et pliable, comprenant une table d'opération (1) dotée d'une dimension longitudinale, d'une dimension transversale et d'une dimension verticale, dans laquelle des dispositifs de commande de guidage de sens X sont agencés par paires sur les deux côtés longitudinaux de la table d'opération (1) ; des bras oscillants principaux (3) sont raccordés à la table d'opération (1) via une base de raccordement principale (2) ; une tige de poussée principale (25) est installée sur la base de raccordement principale (2) ; la tige de poussée principale (25) est raccordée à une terminaison inférieure du bras oscillant principal (3) ; la base de raccordement principale (2) et une terminaison supérieure du bras oscillant principal (3) sont raccordées à un verrou de positionnement principal (5) ; le bras oscillant principal (3) est en outre raccordé à un dispositif de guidage de sens Y (4) ; et le dispositif de guidage de sens Y (4) est raccordé à un dispositif de guidage de sens Z, dans lequel un bras mécanique de portique principal est formé, le verrou de positionnement principal (5) verrouille et soutient le bras mécanique de portique principal ;
des bras oscillants secondaires (6) sont raccordés à la table d'opération (1) via une base de raccordement secondaire installée sur les dispositifs de commande de guidage de sens X, et une traverse (7) est raccordée entre les bras oscillants secondaires (6) via un mécanisme de changement rapide (27), dans lequel un deuxième bras mécanique de portique est formé ; et
une deuxième tige de poussée (26) est raccordée entre la base de raccordement secondaire et le bras oscillant secondaire (6) ; la deuxième tige de poussée (26) est raccordée à une terminaison inférieure du bras oscillant secondaire (6) ; et un deuxième verrou de positionnement (8) afin d'accroître la rigidité du bras oscillant secondaire (6) est agencé entre le bras oscillant secondaire (6) et la base de raccordement secondaire de sorte que le deuxième verrou de positionnement (8) verrouille et soutient le bras mécanique de portique secondaire ; dans lequel la dimension longitudinale de la table d'opération (1) est définie comme sens X ; la dimension transversale de la table d'opération (1) est définie comme sens Y et, la dimension verticale de la table d'opération (1) est définie comme sens Z ; le cadre de référence X, Y, Z formé par le sens X, le sens Y, et le sens Z est orthogonal ; et **caractérisé en ce que**
la base de raccordement principale (2) est installée sur les dispositifs de commande de guidage de sens X ;
la tige de poussée principale (25) pousse le bras oscillant principal (3) afin qu'il pivote autour d'un axe d'oscillation principal parallèle au sens Y ;
la tige de poussée secondaire (26) pousse le bras oscillant secondaire (6) afin qu'il pivote autour d'un axe d'oscillation secondaire parallèle au sens .

2. Un robot chirurgical à bras mécanique double-portique détachable et pliable selon la revendication 1, dans lequel le dispositif de commande de guidage de sens X comprend un rail de guidage de sens X (10) ; un bloc coulissant principal de sens X (11) et un bloc coulissant secondaire de sens X sont agencés sur le rail de guidage de sens X (10) ; le bloc coulissant principal de sens X (11) est raccordé à la base de raccordement principale (2) ; le bloc coulissant secondaire de sens X est raccordé à la base de raccordement secondaire ; et un moteur à mouvement de sens X (12) est agencé à une terminaison du rail de guidage de sens X (10).

3. Un robot chirurgical à bras mécanique double-portique détachable et pliable selon la revendication 1, dans lequel une terminaison supérieure du bras oscillant principal (3) est raccordée au dispositif de guidage de sens Y (4) via un dispositif d'ouverture et de fermeture ; le dispositif d'ouverture et de fermeture comprend un manchon de positionnement (24) ; le manchon de positionnement (24) est muni d'un trou de montage, et un écrou de verrouillage (20) est agencé dans le trou de montage de manière pénétrante ; le manchon de positionnement (24) est installé au sommet du bras oscillant principal (3) via un boulon de verrouillage ; une protubérance de positionnement (21) est agencée sur le manchon de positionnement (24), une gorge (22) est agencée à la position correspondante du sommet du bras oscillant principal (3), et la protubérance de positionnement (21) est incorporée dans la gorge (22).

4. Un robot chirurgical à bras mécanique double-portique détachable et pliable selon la revendication 1 ou 3, dans lequel le bras oscillant principal (3) est muni d'une gorge principale de logement (9) pour loger le verrou de positionnement principal (5), et le bras oscillant secondaire (6) est muni d'une gorge de logement secondaire pour loger le verrou de positionnement secondaire (8).

5. Un robot chirurgical à bras mécanique double-portique détachable et pliable selon la revendication 4, dans lequel le verrou de positionnement principal (5) et le verrou de positionnement secondaire (8) comprennent chacun un manchon de raccordement (13) ; une tige de raccordement supérieur (15) est insérée dans la terminaison supérieure du manchon de raccordement (13), et une tige de raccordement inférieur (14) est insérée dans la terminaison inférieure du manchon de raccordement (13) ; une terminaison supérieure de l'intérieur du manchon de raccordement (13) est munie d'un filetage à droite principal, et une terminaison inférieure de l'intérieur du manchon de raccordement (13) est munie d'un filetage à gauche principal ; une partie de la tige de raccordement supérieur (15) insérée dans le manchon de raccordement (13) est munie d'un filetage à droite principal, et une partie de la tige de raccordement inférieur (14) insérée dans le manchon de raccordement (13) est munie d'un filetage à gauche principal; et un écrou à rotations avant et arrière est agencé dans le manchon de raccordement (13).

6. Un robot chirurgical à bras mécanique double-portique détachable et pliable selon la revendication 4, dans lequel une boucle de raccordement (23) est agencée au sommet du bras oscillant secondaire (6), et la traverse (7) effectue positionnement et raccordement via la boucle de raccordement (23).

7. Un robot chirurgical à bras mécanique double-portique détachable et pliable selon la revendication 1 ou 3, dans lequel le dispositif de guidage de sens Y (4) comprend un moteur d'entraînement de sens Y (16) ; un arbre rotatif (17) est installé sur le moteur d'entraînement de sens Y (16) ; et la tige de poussée principale (25) est raccordée entre la base de raccordement principale (2) et le bras oscillant principal (3).

8. Un robot chirurgical à bras mécanique double-portique détachable et pliable selon la revendication 1, dans lequel un capteur télémétrique laser (28) est installé sur le dispositif de commande de sens Z ; un dispositif de fixation articulaire est installé sur la traverse (7) ; le dispositif de fixation articulaire comprend une plaque de fixation (29) ; un indicateur de niveau (30) est installé sur la plaque de fixation (29) ; et la plaque de fixation (29) est simultanément raccordée à une aiguille principale d'acquisition de coordonnées (31) et une aiguille secondaire d'acquisition de coordonnées (32).

9. Un robot chirurgical à bras mécanique double-portique détachable et pliable selon la revendication 8, dans lequel la plaque de fixation (29) est en forme de U ; la plaque de fixation (29) est munie d'un trou de fixation, et un clou de fixation est raccordé au trou de fixation ; une tige principale de fixation (33) et une tige secondaire de fixation (34) sont agencées sur la plaque de fixation (29), respectivement ; l'aiguille principale d'acquisition de coordonnées (31) est installée sur la tige principale de fixation (33), et l'aiguille secondaire d'acquisition de coordonnées (32) est raccordée à la tige secondaire de fixation (34).

10. Un robot chirurgical à bras mécanique double-portique détachable et pliable selon la revendication 8, dans lequel l'aiguille principale d'acquisition de coordonnées (31) et l'aiguille secondaire d'acquisition de coordonnées (32) comprennent chacune une canule accommodante (35) ; une pluralité de corps d'aiguilles (36) est distribuée en rangée dans la canule accommodante (35) ; et une structure de traversée d'arbre (37) est agencée sur un côté de la canule accommodante (35).

11. Un robot chirurgical à bras mécanique double-portique détachable et pliable selon la revendication 1 ou 8, dans lequel le dispositif de commande de sens Z comprend un moteur d'entraînement de sens Z (18), et un mécanisme de changement rapide (19) est installé sur le moteur d'entraînement de sens Z (18).

12. Un robot chirurgical à bras mécanique double-portique détachable et pliable selon la revendication 1, dans lequel une bibliothèque d'instruments à tambour rotatif et un mécanisme d'entraînement rotatif (38) est installé sur le bras mécanique de portique secondaire, et comprend un tambour rotatif (39) ; une table rotative (40) est agencée à la base du tambour rotatif (39) ; un mécanisme de levage orthopédique (41), un mécanisme de levage d'outil de coupe de tissus mous (42) et un mécanisme de levage de pinces (43) sont agencés dans le tambour rotatif (39), respectivement ; une terminaison supérieure du tambour rotatif (39) forme une chambre de moteur (44), et une terminaison inférieure du tambour rotatif (39) forme une chambre de transmission (45).

13. Un robot chirurgical à bras mécanique double-portique détachable et pliable selon la revendication 12, dans lequel le mécanisme de levage orthopédique (41) comprend un premier moteur (46) situé dans la chambre de moteur (44) ; et une terminaison inférieure du premier moteur (46) est raccordée à un premier rail de guidage (47) situé dans la chambre de transmission (45) ; un premier bloc coulissant (48) est installé sur le premier rail de guidage (47), et un connecteur électrique orthopédique (49) est installé sur le premier bloc coulissant (48).

14. Un robot chirurgical à bras mécanique double-portique détachable et pliable selon la revendication 12, dans lequel le mécanisme de levage d'outil de coupe de tissus mous (42) comprend un deuxième moteur (50) situé dans la chambre de moteur (44) ; une terminaison inférieure du deuxième moteur (50) est raccordée à un deuxième rail de guidage (51) situé dans la chambre de transmission (45) ; un deuxième bloc coulissant (52) est installé sur le deuxième rail de guidage (51), et un connecteur d'outil de coupe de tissus mous (53) est installé sur le deuxième bloc coulissant (52).

15. Un robot chirurgical à bras mécanique double-portique détachable et pliable selon la revendication 12, dans lequel le mécanisme de levage de pinces (43) comprend un troisième moteur (54) situé dans la chambre de moteur (44) ; une terminaison inférieure du troisième moteur (54) est raccordée au troisième rail de guidage (55) situé dans la chambre de transmission (45) ; un troisième bloc coulissant (56) est installé sur le troisième rail de guidage (55), et un connecteur de pinces (57) est installé sur le troisième bloc coulissant (56).
